# EUROPEAN PATENT SPECIFICATION

(11) **EP 0 874 985 B1**
(45) Date of publication and mention of the grant of the patent: **07.05.2003**
(21) Application number: 96941479.6
(22) Date of filing: 26.11.1996
(51) Int. Cl.: G01N 33/483, G01N 33/50, A01C 7/04

(54) **AGRICULTURAL PRODUCT MICROSCREEN METHOD AND APPARATUS**
VERFAHREN UND VORRICHTUNG ZUR ÜBERPRÜFUNG VON LANDWIRTSCHAFTLICHEN PRODUKTEN IN KLEINEM MASSSTAB
PROCEDE ET APPAREIL DE MICROCRIBLAGE DE PRODUITS AGRICOLES

(30) Priority: 01.12.1995 US 7851 P
(43) Date of publication of application: 04.11.1998
(73) Proprietor: E.I. DU PONT DE NEMOURS AND COMPANY, Wilmington Delaware 19898 (US)
(72) Inventor: BHIDE, Arvind, Krishna, Newark, DE 19711 (US); KOEPPE, Mary, Kolean, Landenberg, PA 19350-9600 (US); CASPAR, Timothy, Yorklyn, DE 19736-9702 (US); DIEHL, Kevin, Eugene, Newark, DE 19711-4839 (US); BUSH, Carol, L., Rising Sun, MD 21911-2631 (US); FRENTZEL, Theodore, William, Jr., Newark, DE 19711-3767 (US); ROSANIO, Louis, G., Jr., Wilmington, DE 19810-2245 (US)
(74) Representative: Beacham, Annabel Rose
(86) International application number: US9618915
(87) International publication number: WO97020209

(56) References cited:
- EP-A- 0 258 565
- WO-A-93/25067
- FR-A- 2 661 068
- FR-A- 2 661 799
- GB-A- 2 048 035
- GB-A- 2 155 296
- US-A- 3 750 832
- US-A- 4 401 236
- US-A- 4 402 635
- US-A- 4 897 345
- DATABASE WPI Week 9502 Derwent Publications Ltd., London, GB; AN 1995-008995 XP002027412 & JP 06 292 461 A (JAPAN TOBACCO INC), 21 October 1994
- DATABASE WPI Week 8413 Derwent Publications Ltd., London, GB; AN 1984-080089 XP002027413 & SU 1 020 067 A (UKR PLANT PROTECT), 30 May 1983
- DATABASE WPI Week 8701 Derwent Publications Ltd., London, GB; AN 1987-005786 XP002027414 & SU 1 230 485 A (CORN RES INST), 5 January 1986

## Description

### BACKGROUND OF THE INVENTION

Herbicide candidates are normally identified either by screening test compounds *for* biological activity on intact plants grown in soil ("greenhouse" assays) or by screening test compounds for inhibition of specific target enzymes or other proteins *in vitro* ("*in vitro"* assays). In greenhouse assays, test compounds are either sprayed onto soil containing ungerminated seeds (pre-emergent application) or onto the plants themselves (post-emergent application). Greenhouse assays have a high success rate in predicting the herbicidal activity of a compound when applied in the field. Additionally, a greenhouse assay simultaneously tests for compounds which affect essentially all potential modes of herbicidal action In other words, greenhouse assays possess a high information content. However, greenhouse testing is time- and space-consuming, requiring 2 weeks and 2 ft² of greenhouse space for each compound tested. Large amounts of labor are also required to prepare, care for, spray and score these tests. Moreover, 10 to 60 mg of a test compound is required to assess its potential herbicidal activity at normal use rates. The requirement for this amount of test compound places significant limitations on the synthesis or acquisition of compounds to be tested.

*In vitro* assays, by contrast, typically assay the affect of a test compound on only a specific target enzyme or protein. These targets are usually extracted proteins, and their responses to the test compounds are assayed *in vitro*. Alternatively, these targets are expressed in surrogate microorganisms and their responses are assayed by their effects on the growth or metabolism of the microorganisms. In either case, these assays have great advantages over greenhouse assays, including much lower space, labor, time and compound requirements. However, the value of these assays for predicting herbicidal activity under natural conditions is limited by complex and poorly understood processes unique to intact plants which are absent *in vitro* or in surrogate organisms. Processes such as uptake, translocation, and metabolism of test compounds cannot at present be accurately predicted and must be determined empirically by evaluating responses on intact plants. Moreover, *in vitro* screens are only capable of screening test compounds against a single or very small number of targets, thus requiring separate assays for each new target.

A method for screening compounds for herbicidal activity which combines the high information content and good predictive qualities of greenhouse screening with the reduced size, cost and compound requirements of *in vitro* screens, wherein high-throughput screening of test compounds can be accomplished using whole plant responses as the assay, is desirable.

Dornbos and Spencer have examined the germination and early seedling growth of three species of weeds (*Medicago sativa, Lolium multiflorum,* and *Abutilon theophrasti*) in 24-well tissue culture plates (Dornbos, D. L. Jr. and Spencer, G. F. *J. Chem. Ecol.* (1990), *16,* 339-351). The wells in these plates were cylinders, about 1.6 cm in diameter and 1.7 cm in height, with a total volume of about 3.4 cm³. Each well contained 1 mL of 0.5% agar in water. Various amounts of test compounds were added to the surface of the agar in 1 mL of solvent (99% hexane, 1% chloroform) and the solvent was then allowed to evaporate. Seeds were placed on the agar and after 3 days, seed germination and seedling length were measured. This assay is terminated at 3 days after inoculation of the seed onto the agar, a point at which the seedlings are still largely dependent on seed reserves for growth. Thus, it is likely that the short term growth will not allow the detection of injury of certain classes of herbicides which affect photosynthetic or other processes which are not required for early seedling growth.

GB-A 2048035 discloses a device for sowing seeds in a predetermined pattern, the device having a manifold in the form of a seed head which has a seed plate in which a plurality of apertures are formed. The manifold is adapted to communicate alternately with a source of air under pressure and a source of suction. When the manifold is under the influence of suction a seed can be retained against each aperture and when the manifold is under the influence of air pressure, air will pass through the apertures to eject seeds from the seed head.

US 3,750,832 discloses seed lifters for use in the mechanical sowing of a number of seeds which are distributed to a predetermined number of sowing places with a predetermined number of seeds per sowing place. Each of the seed lifters includes a suction nozzle having a plurality of spaced openings operatively connected alternately to the suction side and the pressure side of an air compressor. The diameter of the openings is considerably less than the size of the seed to be planted.

### SUMMARY OF THE INVENTION

This invention pertains to a method of automatically depositing a seed into each of a plurality of containers, each container having a cross-sectional area of not greater than 1 cm² and each seed being one that will germinate into a plant of the genus selected from the group consisting of *Ageratum*, *Arabidopsis, Arabis*, *Artemisia, Asrostis*, *Browallia, Capsella, Coleus, Cortaderia, Cynodon, Cyperus, Digitalis, Digitaria, Eragrostis, Inula, Ipomopsis, Laevis, Lemna, Nicotiana, Oxalis, Panicum, Petunia, Platycodon, Recta, Sagina, Santolina, Thymophylla, and Thymus,* the method comprising providing the seeds in at least one fluidized bed, providing a plurality of vertical hollow seed transport tubes each of such tubes having at its lower end an opening the largest dimension of which is smaller than the smallest dimensions of the seed, each of said tubes having means for controlling the air pressure within the hollow portion of the tube, the air pressure within each tube being individually controllable, positioning the plurality of tubes relative to the at least one fluidized seed beds such that the lower ends of the plurality of tubes is partially immersed in the at least one fluidized seed bed, controlling the air pressure within each tube to create a vacuum within each tube sufficient to capture and hold a seed at the opening at the lower end of each tube, positioning the plurality of tubes relative to the containers such that the lower end of each tube is within a container, positioned approximately in the center of the horizontal cross section of each container, below the level of the top of the container and above the level of any contents in the container, controlling the air pressure within each tube to release the vacuum and create pressure within each tube higher than the pressure outside each tube sufficient to release and expel the seed into the container, and automatically detecting whether one and only one seed has been placed in a predetermined target area in each of the plurality of containers, and recording which containers have not had one seed so placed. Preferred is a method wherein the at least one seed is a seed which will germinate into a plant of the genus selected from the group consisting of *Arabidopsis*, *Asrostis*, *Browallia, Coleus, Digitalis, Digitaria*, *Eragrostis*, *Inula, Nicotiana, Oxalis, Panicum* and *Petunia.* Most preferred for use in the method is a seed which will germinate into a plant selected from the group consisting of *Arabidopsis thaliana, Browallia speciosa, Coleus blumei, Digitaria sanguinalis, Nicotiana tobacum,* and *Petunia hybrida.*

Each seed may be in contact with a plant growth medium and a compound to be evaluated, each container being maintained under conditions such that in the absence of the compound the seed would germinate into a single plant and the resulting plant would grow in the container.

The method further comprises having in each container before the seed is expelled therein a substance which when contacted by the seed will inhibit or prevent further movement of the seed within the container and inhibit or prevent the seed from being expelled from the container due to air currents resulting from any excess air pressure within the tube.

The method of the present invention also comprises detecting and recording whether each container has had placed therein one and only one seed, and optionally correcting the seeding of any container found not to contain one and only one seed. The method further comprises such detecting being done automatically by optical scanning techniques.

The present invention further comprises apparatus for carrying out the above-described method, which apparatus is described in greater detail below.

### BRIEF DESCRIPTION OF THE FIGURES

Figure 1 is a schematic elevation view of the overall automated seeding system.
Figure 2A is an isometric view of the seed handling and monitoring system.
Figure 2B is a break away of a portion of the seed handling system.
Figure 3 is a section view of a seed pickup tube.
Figure 4 is an enlarged view of a row of wells in a microtiter plate as seen by a monitoring camera.
Figure 5 is an isometric view of the well illumination and correction station.
Figure 6 is a partial schematic illustration of pneumatics for the seed pickup tubes.
Figures 7A and 7B are a perspective view and section view, respectively, of a microtiter lid and egg pickup tube for use in egg placement.

### DETAILED DESCRIPTION OF THE INVENTION

The apparatus of the invention may be used in a method for screening compounds (including both synthetic chemical compounds and compounds occurring in nature) for herbicidal activity (termed the herbicide microscreen) which combines the high information content and good predictive qualities of greenhouse screening with the reduced size, cost and compound requirements of *in vitro* screens. This screening method employs intact plants grown in small containers on very small amounts of growth media containing the test compound In comparison to the standard greenhouse screen, the microscreen requires vastly less space, labor, and test compound. However, unlike *in vitro* screens, responses of intact plants are assayed. Using the microscreen, high-throughput screening of test compounds can be accomplished using whole plant responses as the assay. This method for detection of compounds and molecules that display herbicidal activity is capable of screening large numbers of candidates in a format that minimizes space, materials, waste stream and labor costs.

In a preferred embodiment, a plurality of containers of substantially the same dimension as each other is arranged in a rectangular matrix on a single supporting plate, and the method comprises placing into each of the plurality of containers a growth medium, at least one seed and either at least one compound to be evaluated or a control material. The method exploits a physical format of testing vessels that is in common use in research laboratories and clinical settings. In the most preferred embodiment, this format, commonly referred to as a microtiter plate, generally employs a test unit comprised of a series of wells, known as microwells, wherein the footprint, or the two dimensional space that the unit occupies, is approximately 9 cm by 13 cm. This format is especially convenient in that many instruments have been developed (and are commercially available) that automate plate movement, well filling, aspirating and washing, and data collection from individual microwells. The opening of each microwell of the microtiter plates contemplated by this invention can be square or, preferably, circular, generally with a cross-sectional area of less than 1 cm², prefereably less than 0.5 cm². Although a "standard" microtiter plate is comprised of cylindrical microwells arranged in an 8 x 12 matrix that are approximately 1 cm deep and can accommodate a liquid volume of 300-400 uL, this invention also pertains to other microtiter plates wherein the microwells are deeper ("deep well" microtiter plates) and can therefore accommodate a larger fluid volume, or alternatively wherein there are a greater number of wells per plate (for example, Nunc 384 Well Plate; Cat. No. 242757; Nunc, Inc., Naperville, IL). These other microtiter plates maintain the described 9 x 13cm footprint, thus facilitating automated handling of microtiter plates in support of the high-throughput nature of the screening method. Deeper wells accommodating a larger fluid volume may be employed in order to afford growth of certain plant species; greater numbers of wells per plate will increase the number of compounds that can be screened per assay plate and reduce the quantities of materials (compounds, growth media) consumed per assay.

### Species Selection

Unless otherwise specified, the experiments described herein were conducted under the following conditions. One hundred microliters of molten 0.4% agarose in 1/2x MS salt media without sugar and vitamins (Murashige, T. and Skoog, F. *Physiol*, *Plant.* (1962) *15,* 485; hereinafter "1/2x MS") were pipetted into each well of 96 well microtiter plates, allowed to cool and harden, and one seed per well was placed on the top of the agarose using a forceps. The microtiter plates were sealed with gas permeable tape to prevent the growth media from drying out and the plates were placed in an incubator at 25°C/21°C day/night, 16 h photoperiod (45-80 µmol/m2/s photosynthetically active radiation (PAR)) and 60-90% relative humidity. Plates were kept in plexiglass boxes and were visually rated 14 days after planting (DAP). CO₂ concentration was maintained at an artificially high level (approximately 1%) by daily addition of a 1.5 g piece of dry ice to the plexiglass boxes housing the microtiter plates.

A total of 192 species were tested for growth in 96-well microtiter plates. Species were selected for testing based primarily on the small size of their seeds. Seeds were purchased from Germania (Chicago, IL) or were propagated at Stine Research Center (DuPont Co, Newark, DE). Plates were prepared as described above and 24-32 seeds were planted per species. Germination rate and growth habits of each species were observed visually 7 and 14 DAP. All species evaluated were compared using, primarily, ratings (described below) and general growth characteristics. Those species exhibiting appropriate size, shape and uniformity were advanced for additional testing. Germination rate was a factor when species within a single genus were compared. The rating scale used was as follows:

| | |
|---|---|
| Excellent | Ideal plant for microtiter plate growth, strong in all categories (size, shape, uniformity). |
| Good | Nearly ideal, but lacking in one or two categories, weaknesses can probably be overcome. |
| Poor | Will grow, but has glaring weaknesses which may be difficult to overcome. |
| Unacceptable | Will not work in microtiter plates. |

Of the 192 species tested, a total of 68 species were advanced for further testing in ambient and high CO₂ (1%) environments. Plants held in a high CO₂ environment were grown in sealed plexiglass boxes and 1.5 g dry ice was added daily to achieve the 1% CO₂ level. From this test, 35 species (listed in Table 1) were selected which were rated good to excellent as previously described.

**Table 1.**

| Plant species selected for testing biological response to commercial herbicides. | | |
|---|---|---|
| Genus | Species | Common Name |
| *Ageratum* | sp. | *Ageratum* (blue danube) |
| *Arabidopsis* | *thaliana* | *Arabidosis* |
| *Arabis* | *blepharophylla caucasica* | *Arabis* (compinkie) |
| *Artemisia* | *dracunculus* | Tarragon |
| *Asrostis* | *stolonifera* | Bentgrass |
| *Browallia* | *speciosa* | *Browallia* (marine bells) |
| *Browallia* | *speciosa* | *Browallia* (jingle bell) |
| *Capsella* | *bursa-pastoris* | Shepherdspurse |
| *Coleus* | *blumei* | *Coleus* (wizard golden) |
| *Coleus* | *blumei* | *Coleus* (rainbow-strain) |
| *Coleus* | *blumei* | *Coleus* (pink dragon) |
| *Cortaderia* | *selloana-pink* | Pampus Grass |
| *Cynodon* | *dactylon* | Bermudagrass |
| *Cyperus* | *difformis* | *Cyperus* |
| *Digitalis* | *purpurea* | *Digitalis* (white) |
| *Digitalis* | *purpurea* | *Digitalis* (excelsior) |
| *Digitaria* | *sanguinalis* | Large crabgrass |
| *Eragrostis* | *curbula* | Weeping lovegrass |
| *Inula* | *ensifolia* | Ensifolia |
| *Ipomopsis* | *elegans* | Capitata Gilla |
| *Laevis* | *jasione-perennis* | Perennis Jasione |
| *Lemna* | *minor* | *Lemna* |
| *Nicotiana* | *tobacum* | Tobacco |
| *Oxalis* | *stricta* | Yellow woodsorrel |
| *Panicum* | *dichotomiflorum* | Fall panicum |
| *Panicum* | *coloratum* | Kleingrass |
| *Petunia* | *hybrida* | *Petunia* (star joy) |
| *Petunia* | *hybrida* | *Petunia* (carpet mixture) |
| *Petunia* | *hybrida* | *Petunia* (velvet frost) |
| *Platycodon* | *grandiflorum* | *Platycodon* |
| *Recta* | *warrensii* | Potentilla |
| *Sagina* | *subulata* | Pilifera spergula |
| *Santolina* | *chamaecyparissus-tomentasa* | Chamaecyparissus |
| *Thymophylla* | *puntuiloba-dyssodia* | Dahlborg daisy |
| *Thymus* | *vulgaris* | Thyme |

### Automation of Seeding

Several methods of automating the seeding have been tested with the goal of placing one seed in each well of a 96-well microtiter plate.

A template seeder was designed by Berry Seeder Co. (Elizabeth City, NC) for seeding into 96-well microtiter plates. This seeder has stainless steel plates with 96 holes which are in alignment with the microtiter plate wells. There were 4 stainless steel plates, each with different diameter holes to accommodate the different sized seeds. Each stainless steel plate is attached to a plexiglass box containing a vacuum line. High pressure air was run into a separate valve in the box and opening the valve resulted in vibration of the stainless steel plate. Seeds were placed on the stainless steel plates with the vacuum running and the high pressure air valve opened to vibrate excess seeds out of each hole. Experiments were run with *Arabidopsis thaliana, Browallia speciosa, Coleus blumei, Digitalis purpurea, Digitaria sanguinalis*, *Nicotiana tobacum* and *Petunia hybrida*. The Berry template seeder worked well with *Browallia* and *Coleus* seeds with 90-95% singulation. These seeds are quite round and were the largest seeds tested (0.040" diameter for *Coleus* and 0.035" diameter for *Browallia*). *Digitaria,* which is an elongated seed, could not be singulated because it would not stick in the holes (holes were too small). With *Petunia* and *Digitalis* seeds, the holes in the stainless steel plates were either too large (seeds were pulled through the holes) or too small (seeds would not stick). *Arabidopsis* seeds had multiple seeds in many of the holes. *Nicotiana* seeds had 26-45% singulation with the remainder being either multiple seeds or no seeds (seeds would be pulled through the holes). Although the Berry template seeder had high singulation of the *Coleus* and *Browallia* seeds, the seeder is quite temperamental. It takes some "tweaking" to attain this level of singulation. This seeder did not work well with the other seeds. Based on these tests, this seeder would not work for automation of seeding.

A needle seeder (Seed-Air-Matic™, KW Engineering Pty. Ltd., Queensland, Australia) for seeding into plug trays has been tested to determine the feasibility of using with the following seeds: *Arabidopsis*, *Browallia, Coleus, Digitaria, Nicotiana, Oxalis* and *Petunia.* This seeder has 8-20 syringe needles of varying bore sizes that were connected to vacuum. Seeds were placed in a hopper that vibrates. The needles dipped into the hopper, picked up individual seeds and dropped the seeds into guide tubes, which in turn released into plug trays using a short burst of high pressure air. A row of seeds was picked up and dropped in 1-4 seconds. The KW Engineering seeder was used to test singulation of *Arabidopsis*, *Browallia, Coleus, Digitaria, Nicotiana, Oxalis* and *Petunia* seeds into 96-well microtiter plates. This seeder has been modified to seed into microtiter plate format. *Browallia*, *Coleus, Digitaria, Nicotiana, Oxalis* and *Petunia* seeds had 90-96% singulation using a single needle. *Arabidopsis* singulation rate was about 83-85%. Individual vacuum regulators have been installed to achieve these levels of singulation with multiple needles (8-12). At these singulation rates, missed or multiple seeded wells can be corrected manually.

Batching Systems (Owings, MD) uses a vibratory bowl to singulate parts (nuts, bolts, game pieces, etc.) and an optical eye to count the parts. For use as a seeder specifically for seeding into 96- or 48-well microtiter plates, seeds would be placed in a vibratory bowl and would "walk" up a ramp. At the end of the ramp, the width would be adjusted so that only one seed at a time could fit on the ramp. The seeds would drop down and be counted by a dual-view optics system. A microtiter plate would be placed on an X-Y table to singulate seeds.

The method of the invention utilizes a combination of elements making up a seed dispensing system that features a seed pick and place mechanism for a plurality of containers, a seed placement monitoring device for a plurality of containers, and a control system to inform the operator of the location of potentially misplaced seeds or to provide feedback for automatic re-seeding of any missed containers. Alternatively, a discrete correction station may be a component of the system. The pick and place mechanism comprises the feature of individual control of vacuum to individual seed holding tubes (or needles). This individual control of vacuum combined with the seed pickup tube design improves the reliablity for single seed pickup. Additional features of the preferred method include the proper positioning of tip of each seed holding tube during seed pickup and during seed deposition, and providing in each container a substance that when contacted with the seed will inhibit or prevent further movement of the seed within the container and inhibit or prevent the seed from being expelled from the container due to air currents and/or static charge.

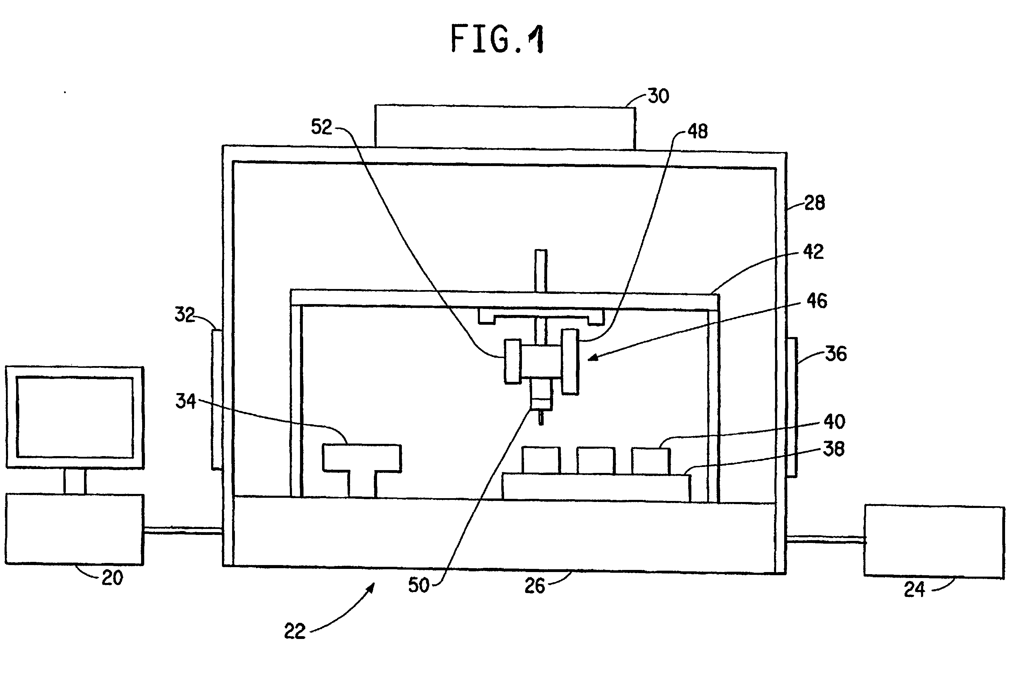

Figure 1 shows the schematics of the basic elements of an automated system for placing seeds into wells of a plurality of microtiter plates, monitoring the accuracy of placement, and correcting for errors. The system will also work to place insect eggs into microwells so as to enable an automated insecticide micro-assay, although the system will be described in the context of handling seeds for an automated herbicide micro-assay. A control device 20 is connected to a seed handling and monitoring device 22 and a seed correcting station 24. The seed handling and monitoring device consists of a base 26 covered by an enclosure 28 which may be supplied with positive pressure filtered air by air supply device 30 or may be provided with negative pressure to control volatile organic compounds. Access door 32 allows access for placing seeds in a seed supply station 34, and access door 36 allows access for loading and unloading a plate carrier 38 with a plurality of microtiter plates, such as plate 40. Within the enclosure is a transport mechanism 42 for transporting an overhead module 46 that contains a monitoring camera 48, a seed holder 50 and a plate identification reader 52.

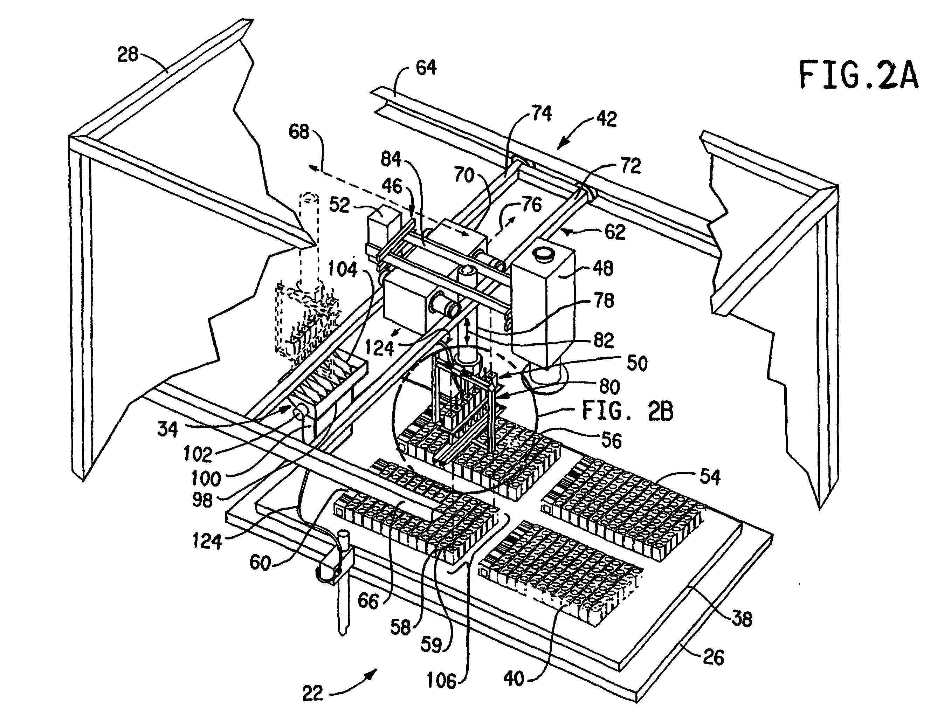

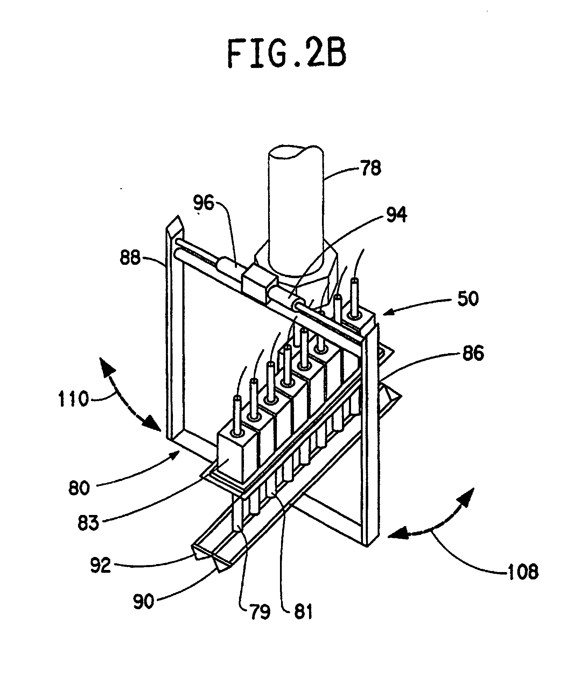

Figure 2A shows an detailed view of the seed handling and monitoring device 22, and Figure 2B shows a break away of a portion of the seed handling system. The base 26 supports the removable plate carrier 38 that is shown with a plurality (4) of microtiter plates 40, 54, 56, and 58 in predetermined locations. The microtiter plates are of the size that contain 96 wells, such as well 59 in plate 58, in a 12X8 array and each plate has a bar code label, such as label 60 on plate 58, preferably on the top surface of the plate. Plates having different dimensions and different numbers and configurations of wells can also be used by making appropriate changes in the seed holder 50. Within each well is a premeasured volume (not shown) of a compound to be tested and a plant growth medium, which can also contain agar. There may be a different compound in each well and several control wells in each plate. Different plates may contain the same compounds to check reproducibility. The carrier can be configured to hold more or less than the illustrated 4 plates. The carrier may be placed on the base either manually, as described via door 36, after removing a lid (not shown) from each plate, or the carrier may be placed automatically from an autofeeder that may hold up to 15 carriers and may include a lid handling feature.

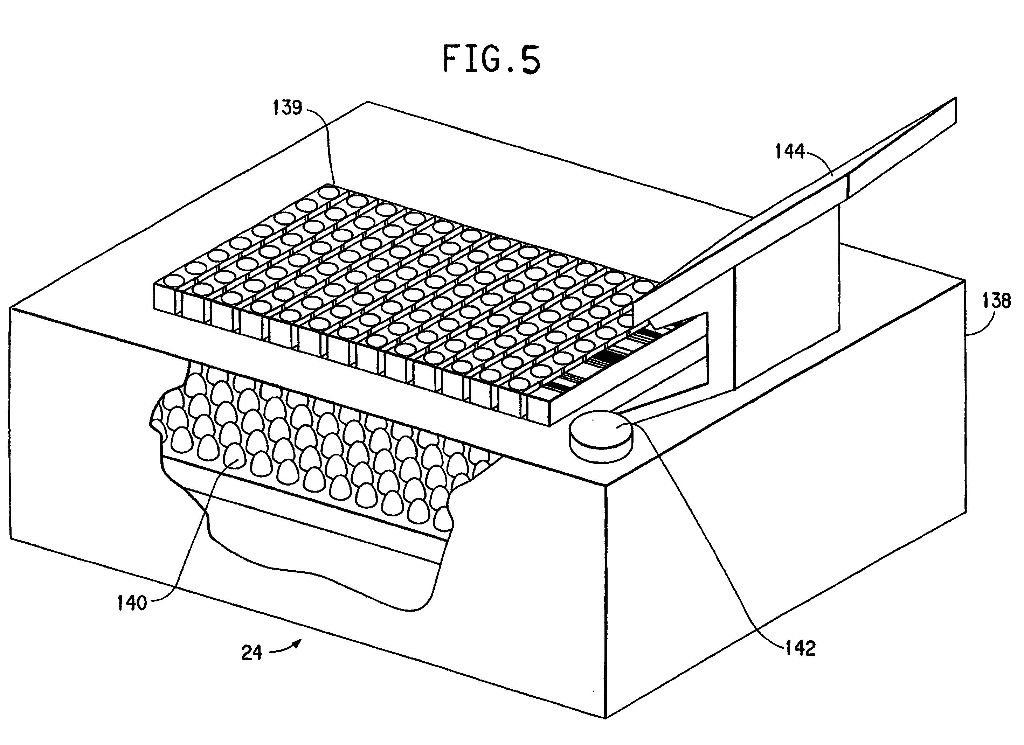

The transport mechanism 42 consists of an overhead X-Y positioning system for overhead module 46, the mechanism comprising a first carriage 62 that runs on rails 64 and 66 for positioning the module in the X-direction indicated by arrows 68; and a second carriage 70 that runs on rails 72 and 74 for positioning the module in the Y-direction indicated by arrows 76. Attached to the carriage 70 is the overhead module 46 that comprises actuator 78, seed holder 50, monitoring camera 48, plate identification reader 52, and seed catcher 80. Actuator 78 mounted on module 46 is for positioning the seed holder 50 in the Z-direction indicated by arrows 82. Elements making up module 46 may be attached to a module frame 84 as shown, or they may be attached directly to carriage 70. The seed holder 50 (Figure 5B) comprises a plurality of seed pickup tubes, such as tube 79 and tube 81 that extend from tube block 83. The seed catcher 80 is attached to actuator 78 and comprises two moveable arms 86 and 88 that support angled bars 90 and 92 respectively that are underneath the ends of the plurality of seed pickup tubes when they are in the upper Z-direction. Actuators 94 and 96 control the motion of arms 86 and 88 respectively.

The seed supply station 34 consists of a seed trough 98 for holding a supply of seeds, a frame 100 resiliently supporting the trough, and a vibrator 102 attached to the trough for vibrating it to cause the seeds to be fluidized or air-borne in the trough. The trough may contain dividers, such as divider 104 to keep the seeds contained in individual compartments in line with individual seed pickup tubes during pickup by seed holder 50. The trough and dividers are preferrably made from a conductive material, such as stainless steel or a filled conductive polymer, that can be grounded to prevent static electricity buildup. The seed supply station may be located in a central position to minimize travel distance to all the wells.

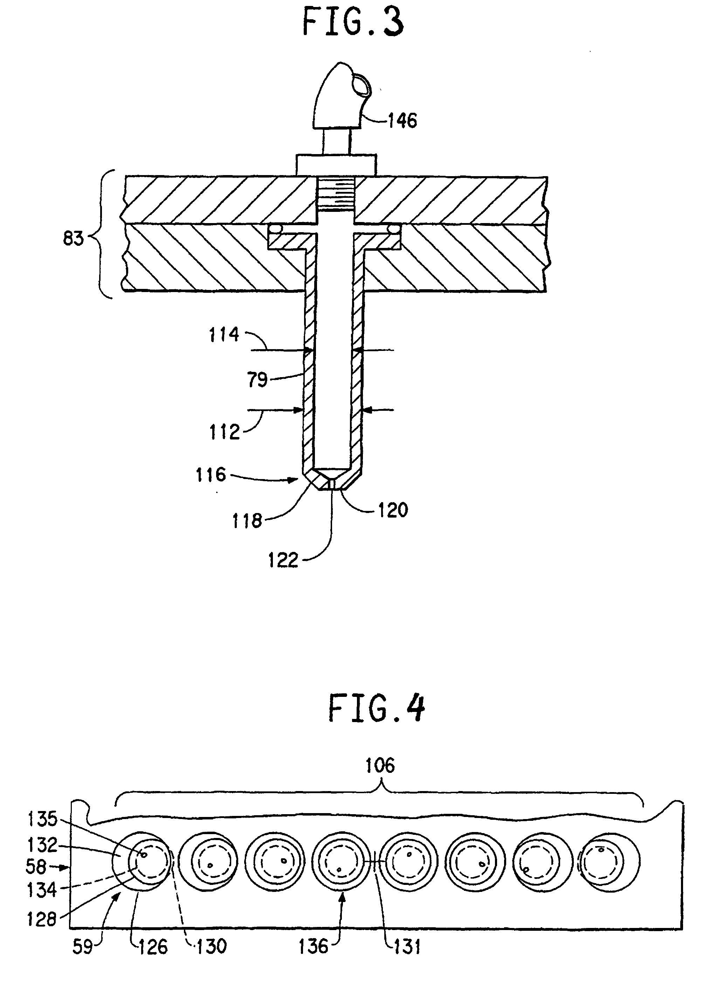

In Figure 2A, the transport mechanism 42 is shown in a position to permit insertion of seeds in a row of wells, such as well 59 in row 106 in plate 58. In this position, plate identification reader 52, also known as a bar code reader, has passed over bar code 60 and identified plate 58 to the control device 20 (Figure 4). There are position encoders (not shown) on the X and Y carriages so the controller 20 can confirm which row of wells the seed holder is positioned over. The seed catcher 80 is in a closed position as shown to catch any seeds that may be dislodged during transport of the seeds from the supply station 34 to the row of wells 106. If a seed were to be dislodged, it would fall into one of the angled bars 90 or 92 instead of landing in one of the wells being crossed during transport, which would result in a seeding error that may not be detected. Periodically, the bars 90 and 92 would be emptied of fallen seeds. Before the seed holder can proceed in the Z-direction to place the seeds in a row of wells, the seed catcher must be in an open position with the arms 86 and 88 moved in the direction of arrows 108 and 110 respectively. This will allow the tube block 83 to pass between bar 90 and bar 92 so that the tips of tubes, such as 79 and 81 can enter the wells, such as 59, in row 106 and deposit in each well of row 106 a seed held by each tube. After deposit of the seeds, the tube block would be retracted in the Z-direction and the transport mechanism 42 would move to place camera 48 over row 106 so each well in row 106 could be monitored for the presence or absence of seeds. The camera 48 is positioned far enough above the wells that its field of view includes all 8 wells in row 106 and the wells are shallow enough at that position so the image of the outermost wells still enables the camera to see the bottom of the well where the seed is deposited. By "bottom of the well" is meant the top surface of the plant growth medium containing compound that is placed in the well and is the surface on which the seed will rest. Typically, in a 10 mm deep well there may be about a 4 mm deep layer of plant growth medium containing compound so the "bottom of the well" is about 6 mm down from the top of the well.

Seed holder 50 is shown with a linear array of 8 tubes, such as 79 and 81, in block 83. This could alternatively be a rectangular array of tubes, such as a 4X2 or 4X4 array, or any other similar arrangement. A more compact array may have an advantage to make monitoring of the wells by camera 48 more reliable by minimizing any parallax optical problems or allowing the camera to be located closer to the wells.

Referring to Figure 3, which is an enlarged section view of tube 79, the tube has a relatively large outer diameter 112 of about 1-4 mm, preferably about 3.0 mm to resist bending and enable accurate positioning in the well. Throughout most of the length of tube 79 there is an enlarged bore 114 of 0.5-3 mm, preferably of about 1.5 mm that terminates near the tip 116. The tip may have a chamfer 118 to present a small flat end 120 to the seeds and deflect adjacent seeds, in motion in a fluidized bed of seeds, away from the single one held at the flat end. A relatively small diameter hole 122 extends from the end of bore 114 through the tip 116 for fluid communication with a single seed. The size of the hole is approximately proportional to the size of the seed. Conveniently, the hole will be circular in cross-section and the diameter of the hole may be smaller than smallest dimension of the seed. For unusually small seeds, this hole 122 may have a diameter of only 0.05 mm. Bore 114 is in communication with a source of air that alternately may be under vacuum or pressure. The pressure can be useful to clear any debris from the hole 122 and to dislodge the seed. To eliminate problems with static electricity near very small lightweight seeds, tube 79, which is representative of all tubes, is made of a conductive material which is corrosion-resistant, such as 304 stainless steel, and is grounded via the block 83, which is also conductive, and a grounding cable 124 (Figure 2A) or other suitable grounding means.

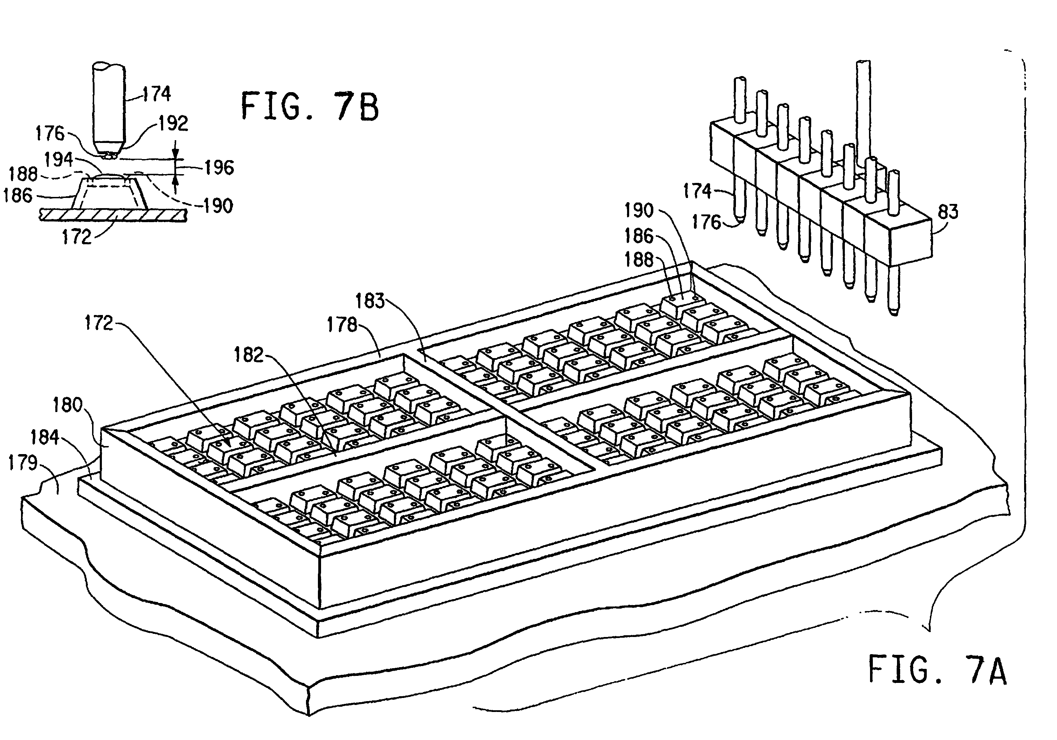

The camera 48 is arranged above the wells at a distance sufficient to obtain a clear high resolution view of the bottom of the wells. Figure 7 illustrates an enlarged perspective view of what the camera sees looking at the row 106 of wells. Circle 126 represents the top of well 59, circle 128 represents the bottom of the well that is a slightly smaller diameter than the top circle 126. Typically, the top surface of plant growth medium containing compound mix will define the visual bottom of the well. Part of the very edge of bottom circle 128 may be partially blocked from view by top circle 126 as is shown at 130. This is due to parallax which is an apparent shift in position of the bottom circle relative to the top circle due to the actual line of sight from the camera center 131 to the well, which is different for each well. Surface 132 represents the sidewall of well 59 that connects the top circle to the bottom circle. Dashed circle 134 represents the target circle for a seed, such as seed 135, placed at the bottom of the well. It is a smaller diameter circle than bottom circle 128 so the seed will be spaced away from the sidewall surface 132. In other wells, such as well 136 near the center of row 106, there may not be any blocking of the bottom circle by the top circle. In the view of Figure 4, the camera can see the entire area of the target circle for all wells. This is important so the camera can notify the control system if the seeds have been successfully placed in the target circle of each well. Based on the camera view, the control system can make several determinations about each seeded well:
1) one seed in the target circle only;
2) no seed in the target circle and no seed in the well;
3) no seed in the target circle and one seed in the well;
4) more than one seed in the well, which may be in or out of the target circle.

A seed that falls at the corner where the bottom circle meets the sidewall may not be seen due to the poor contrast at the corner; and a seed that falls in a blocked area like 130 may not be seen. In such circumstances, there may be a small degree of uncertainty about the determinations. It is expected that such circumstances will be rare due to the accuracy and reliability of the seed placement system. Ideally, such uncertainty is avoided by use of multiple cameras looking at a row or a single camera indexing over two viewing positions, each with fewer wells, to eliminate any parallax problem. Using shallower microtiter wells may also eliminate any parallax problem. The microtiter plates are backlighted to give good lighting and contrast at the bottom of the well for the camera.

If determination #1 is made for all just-seeded wells, the control system can tell the transport mechanism to go get seeds for the next row of wells. If determination #2 is made for any well of the just-seeded wells, the control system can tell the transport mechanism to go get a seed for the particular well that is missing a seed in the target area. If determination #2 persists for the same cell after an attempted correction, the control system can create an error data file to alert the operator that a #2 defect exists for this particular well. If determination #3 is made for any well of the just-seeded wells, the control system can create an error data file to alert the operator that a defect #3 exists for a particular well. If determination #4 is made for any well of the just-seeded wells, the control system can create an error data file to alert the operator that a defect #4 exists for a particular well. The error data file can be used to alert the operator to make manual corrections. In an alternate embodiment, the seed correcting station 24, referring to Figures 1 and 5, is a manually operated/machine assisted station where an operator can place a microtiter plate and examine and correct for determinations #2, 3, and 4 as identified by the camera and control system. It consists of a plate holder 138 that supports a barcoded microtiter plate 139 that a barcode scanner 144 can read. This allows the control device 20 to identify a particular plate. The microtiter plate 139 is over an LED array 140 located within the holder as seen in the cutaway view of Figure 8. The LED array 140 consists of an illuminating LED positioned under each well of the microtiter plate, which in the case shown would be an 8X12 array of 96 LEDs. The control device 20 can independently illuminate particular LEDs by accessing error data files to locate the wells with the #2, 3, and 4 determinations based on the earlier examination of that plate by the monitoring camera 48. The operator can then take the corrective action for each illuminated well. Descriminations between determinations can be made if desired by the manner in which the LED is illuminated, i.e., continuously illuminated, blinking slowly, blinking quickly, etc. The station 24 also has an input switch 142 to permit communication with the control device 20 to turn off selected LEDs, and indicate manual correction has occurred.

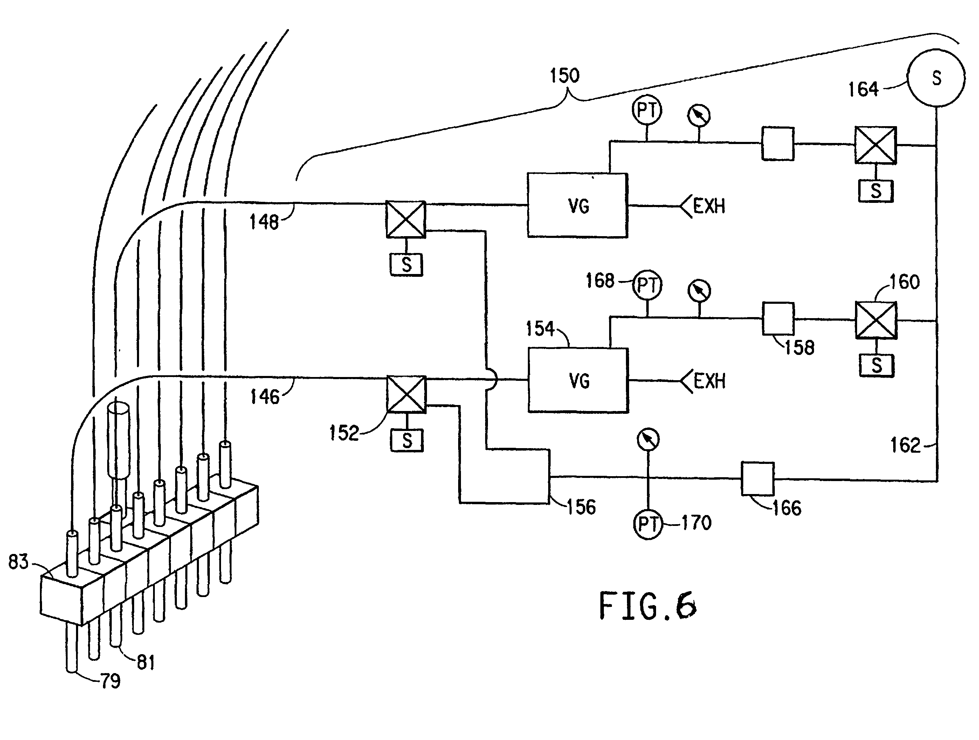

Part of the seed holder 50 is a pneumatic system for controlling the seed pickup and drop off function as seen referring to Figures 9 and 6. Referring to Figure 6, each seed pickup tube, such as tube 79 or 81 in tube block 83, is in fluid communication via conduits 146 and 148, respectively, with pneumatic system 150. This system will be described referring to tube 79 and is understood to be the same for other seed pickup tubes. Conduit 146 is connected to a solenoid operated, three-way, selector valve 152 which is connected to a vacuum generator 154 and a pressure manifold 156. The vacuum generator produces vacuum by a venturi effect and is connected to a pressure regulator 158 and a solenoid operated, two way, selector valve 160, and a pressure manifold 162. The pressure manifold 162 is connected to a source of pressure 164. Pressure manifold 156 is connected to a pressure regulator 166 which is connected to pressure manifold 162. The system allows individual vacuum adjustment to each seed pickup tube for the purpose of 1) adjusting to individual tolerances in the small seed pickup hole, such as hole 122 in tube 79 (Figure 3), and 2) individually turning "on" the vacuum in one or more tubes while it remains "off" in the remaining tubes. The individual vacuum level, or flow, for a tube is individually triggered by actuating the selector valve 160 to direct pressure to the vacuum generator, and then adjusting the pressure regulator 158 to achieve the desired vacuum level. The individual adjustment would be done by trial and error by observing the seed pickup performance for that tube. Alternatively, for automated monitoring of the pneumatic system, there is a pressure transducer 168 between the regulator 158 and vacuum generator 154, and a pressure transducer 170 between pressure regulator 166 and pressure manifold 156. The transducer, such as 168 for each seed pickup tube, and transducer 170 are connected electrically to the control device 20 for continuous monitoring.

During continuous automated operation, the seed handling and monitoring system works as follows referring to Figures 1, 2A, 2B, 3, 5, and 6. Beginning at the position shown in Figure 2A, the operator has already loaded the seeds in the seed supply station 34 and has loaded carrier 38 with microtiter plates 40, 54, 56 and 58 that are prepared with plant growth medium and a different compound for testing in each well, such as well 59. The operator removes the lids from the plates and places the carrier 38 in the enclosure 28 and makes sure the air supply/suction device 30 is providing a steady low flow of filtered air/negative pressure to the enclosure. The operator closes the doors 32 and 36. This minimizes the chance of airborne contamination and controls any air currents that may tend to dislodge seeds during handling. The seed holder 50, as shown in Figure 2B, has already successfully picked up seeds from seed supply station 34 and is positioned over row 106 in preparation for depositing seeds in the wells of row 106.

Automated operation, illustrating reseeding, proceeds as follows:
- valves, such as 160 and 152, remain actuated to maintain vacuum (produced by the vacuum generator 154) and hold a single seed at the tips, such as 116, of all seed holder tubes, such as 79;
- the seed catcher 80 is actuated to move the bars 90 and 92 away from the tips such as 116 of seed holder tubes such as 79;
- actuator 78 is energized to lower the tube block 83 toward the microtiter plate 58 until the tips such as 116 of the tubes such as 79 are inside the wells such as 59 of row 106, which typically places the tips such as 116 about 4 mm away from the bottom of the wells;
- valves, such as 152, are actuated to shift the vacuum "off' and pressure (set at regulator 166) "on" to all the seed holder tubes such as 79 to release the seeds and project them from the tips such as 116 and into each well in row 106, and onto the sticky surface forming the bottom of the wells;
- actuator 78 is energized to raise the tube block 83 back to the upper position and valves, such as 152, are actuated to shift the pressure "off" and the vacuum "on" to tubes such as 79;
- the transport mechanism 42 moves the overhead module 46 to position the camera 48 over row 106 where the seeds were just placed. The camera "reads" the wells and confirms that a single seed has been properly placed in all wells in row 106, except one, and discovers there is no seed in the target and no seed in the bottom of one well in the row, and the control device 20 notes this in an error data file;
- the control device 20 turns "off' the two-way valves 160 to all seed holder tubes except the seed holder tube for the one well that is missing a seed;
- the control device 20 commands the transport mechanism 42 to align the overhead module 46 over the seed supply station 34;
- the actuator 78 is energized to lower the tube block 83 until the tips such as 116 are in the fluidized bed of seeds in the seed trough 98 (for some seeds, the tip is best located just above the upper surface of the fluidized seeds, for other seeds, the tip is best located just at the surface of the fluidized seeds and for some others the tip is located just below the surface of the fluidized seeds);
- the actuator 78 dwells from about 0.01-10 seconds while vacuum is applied to only the one tube to engage a single seed;
- the actuator is energized to raise the tube block 83 back to the upper position;
- the seed catcher 80 is actuated to move the bars 90 and 92 toward the tips such as 116 of seed holder tubes such as 79 to put the bars in position to catch any seeds falling from the ends of the tubes;
- the transport mechanism 42 moves the overhead module 46 over row 106 in plate 58 with the tube block aligned over the row 106;
- the seed catcher 80 is actuated to move the bars 90 and 92 away from the tips such as 116 of seed holder tubes such as 79;
- actuator 78 is energized to lower the tube block 83 toward the microtiter plate 58 until the tips such as 116 of the tubes such as 79 are inside the wells such as 59 of row 106;
- the appropriate valve, such as 152, is actuated to shift the vacuum "off" and pressure "on" to the appropriate seed holder tube 79 to release the seed from the one tube and project it from the tip 116 of that tube and into the well that was previously missing a seed;
- actuator 78 is energized to raise the tube block 83 back to the upper position and the appropriate valve, such as 152, is actuated to shift the pressure "off" and the vacuum "on" to the appropriate tube such as 79;
- the transport mechanism 42 moves the overhead module 46 to position the camera 48 over row 106 where the missing seed was just placed. The camera "reads" the wells and discovers there are now single seeds in the target for all wells;
- the control device 20 turns "on" the two-way valves, such as 160, to all seed holder tubes that had previously been turned "off";
- the control device 20 commands the transport mechanism 42 to align the overhead module 46 over the seed supply station 34;
- after picking up seeds on all tubes such as 79, the control device will command the transport mechanism to position the overhead module over the next row of wells in the microtiter plate and the process will continue until all wells on all plates in the carrier have at least one seed;
- the control device 20 will alert the operator to remove the carriage from the enclosure and notifies the operator about plates that require corrective action;
- the operator places any plate requiring corrective action in the seed correcting station 24;
- the control device identifies the plate with information from bar code scanner 144 and illuminates the LED or LEDs beneath the wells that require corrective action;
- the operator takes corrective action on each well requiring it Upon completing each plate, the operator actuates the input switch 142 to notify the control device 20 to turn off the LEDs to the corrected wells and update the control device data files to indicate manual correction to this microtiter plate is complete.

While the method and apparatus of the present invention is described in detail above with respect to an herbicide assay, this technology can also be used effectively for other assays such as an insecticide assay.

When handling insect eggs with the system just described for seed handling, there are a few variations in the procedure. It may be desired to place the eggs on the inside surface of an elastomeric lid for a microtiter plate, and it may be desired to use a square well microtiter plate that has wells about 4.5 cm (1 3/4") deep so there is adequate space for the insect larve and insect to develop. The elastomeric lids have square protrusions that extend into each square well of the microtiter plate, and each protrusion has two holes in the corners of the protrusion for entry of oxygen for the larve and insect when the lid is in place over the well. A sticky drop of larve support and growth medium is placed on the inside surface or each lid protrusion to hold the eggs in place and support the eggs until the larve hatches. Only lids are placed in the enclosure 28 on the carrier 38 with the inside surface of the lid facing upward. The seed supply station 34 would be used to supply the eggs in much the same way it is used to supply seeds. The overhead module 46 would be much the same for handling and monitoring eggs as for seeds. After placement of the eggs on the lids, the lids are removed from the enclosure and manually placed on the matching microtiter plate for that lid. After hatching, the larve drops into the well and onto the surface of a mix of larve/insect support medium and test compound in the well that fills the well to a height of several millimeters. The effect of the compound on the larve/insect can be observed over a period of time.

Figure 7A shows a partial perspective view of an inverted microtiter lid 172 and egg pickup tubes, such as tube 174, for handling insect eggs, such as 176. If there is a problem with the elastomeric lids distorting when laid free and held only by gravity, one can force them to lay flat for the accurate positioning needed for accurate reliable egg placement, using a weighted frame 178 laid on top of the inverted lid held in place on a carrier 179 (similar to the microtiter plate carrier 38). The frame 178 includes a peripheral rim 180 and central rims 182 and 183 to hold the edges, such as lid edge 184, and central part of the lid, respecively, in a flat condition. The lid 172 has a plurality of protrusions, such as protrusion 186, that are arranged to extend into each well of a microtiter plate (not shown). Each protrusion has a hole in two corners of the protrusion, such as holes 188 and 190 in protrusion 186

Figure 7B shown an enlarged section side view of a protrusion of a lid and an egg pickup tube. One or more eggs 176 are held on the tip 192 of tube 174 by application of vacuum to the tube. A hole (not shown) extending through tip 192, similar to the hole in seed tube 79 of Figure 6, is sized to be a slightly smaller diameter than the minor dimension of a single insect egg, which may have a slightly irregular spherical shape. Because of the irregular shape of the eggs, the vacuum flow can extend around each egg so several eggs can be picked up at one time. The number of eggs picked up can be controlled by adjusting the level of vacuum applied through the hole in tip 192. On top of protrusion 186 is a drop 194 of sticky material for holding the eggs and providing some nutrient for the eggs. When depositing the eggs on top of the protrusion 186, the tip 192 of tube 174 is lowered to within a closely spaced distance 196 of about 4 mm from the surface of the drop so the eggs will be reliably deposited onto the sticky drop when the vacuum is shut off and a pressure is applied to the hole in tip 192. After placing eggs on the top of a row of protrusions, the camera monitor 48 would be positioned over this row of protrusions and would monitor each protrusion in the row to determine if there were some eggs placed there. If there were no eggs present, the control system would tell the transport mechanism to go get eggs for the protrusion missing them, similar to the procedure followed for handling seeds.

Variations in the operation of the seed or egg handling and monitoring system is possible without departing from the basic teachings of the invention. For instance, while normally one and only one seed is desired in each well, there could be circumstances where more than one seed and more than one plant would be desired in each well, such as if the weed being evaluated were sedge, which is extremely small and is a common weed problem in rice crops. In such a circumstance, it would be most convenient to simply repeat the deposition of single seeds the appropriate number of times. Other variations of the invention include different camera monitoring strategies and the use of automated carrier handling. In addition, the carrier may be replaced with a precision indexing conveyor that will accurately move one plate at a time into a seed depositing position under a simplified transport mechanism Plates would be automatically loaded onto the conveyor. The simplified transport mechanism would only move in the X-direction 68 and Z-direction 82, and there would only be two possible X positions. One X position would be with the seed pickup tubes over the seed supply station and at the same position, the camera is over the depositing station monitoring the seed placement. The other X-position would be with the seed pickup tubes over the seed depositing station. The indexing conveyor would position the relevant row of wells at the depositing station. The other wells not at the depositing station would be covered under a shield so the seed catcher 80 could be eliminated.

## Claims

1. A method of automatically depositing a seed into each of a plurality of containers, each container having a cross-sectional area of not greater than 1 cm² and each seed being one that will germinate into a plant of the genus selected from the group consisting of *Ageratum*, *Arabidopsis, Arabis, Artemisia, Asrostis, Browallia, Capsella, Coleus, Cortaderia, Cynodon, Cyperus, Digitalis, Digitaria, Eragrostis, Inula, Ipomopsis, Laevis, Lemna*, *Nicotiana, Oxalis*, *Panicum*, *Petunia, Platycodon, Recta*, *Sagina, Santolina*, *Thymophylla*, *and Thymus,* the method comprising providing the seeds in at least one fluidized bed, providing a plurality of vertical hollow seed transport tubes each of such tubes having at its lower end an opening the largest dimension of which is smaller than the smallest dimensions of the seed, each of said tubes having means for controlling the air pressure within the hollow portion of the tube, the air pressure within each tube being individually controllable, positioning the plurality of tubes relative to the at least one fluidized seed beds such that the lower ends of the plurality of tubes is partially immersed in the at least one fluidized seed bed, controlling the air pressure within each tube to create a vacuum within each tube sufficient to capture and hold a seed at the opening at the lower end of each tube, positioning the plurality of tubes relative to the containers such that the lower end of each tube is within a container, positioned approximately in the center of the horizontal cross section of each container, below the level of the top of the container and above the level of any contents in the container, controlling the air pressure within each tube to release the vacuum and create pressure within each tube higher than the pressure outside each tube sufficient to release and expel the seed into the container, and automatically detecting whether one and only one seed has been placed in a predetermined target area in each of the plurality of containers, and recording which containers have not had one seed so placed.

2. The method of Claim 1 further comprising having in each container before the seed is expelled therein a substance which when contacted by the seed will inhibit or prevent further movement of the seed within the container and inhibit or prevent the seed from being expelled from the container due to air currents resulting from any excess air pressure within the tube.

3. The method of Claim 1 or Claim 2 further comprising correcting the seeding of any container found not to contain one and only one seed.

4. The method of any one of Claims 1 to 3, wherein the cross-sectional area of each container is not greater than 0.5 cm².

5. An apparatus for automatically depositing a plant seed or insect egg into each of a plurality of containers comprising a plurality of vertical hollow seed transport tubes, each of such tubes having at its lower end an opening the largest dimension of which is smaller than the smallest dimension of the seed or egg, each of said tubes having means for individually controlling the air pressure within the hollow portion of the tube, and means for automatically detecting whether one and only one seed or egg has been placed in a predetermined target area in each of the plurality of containers, and for recording which containers have not had one seed or egg so placed.

6. A seed transport for use in the apparatus of Claim 5 comprising a hollow cylindrical tube having an outer diameter of 1-4 mm, an inner bore of 0.5-3 mm, having an opening at the lower end of the tube the largest dimension of which is smaller than the smallest dimension of the seed, and the lower end of the tube having a chamfer to deflect seeds away from a seed which is being held at the opening.

## Patentansprüche

1. Verfahren zum automatischen Ablegen eines Samens in jeden einer Vielzahl von Behältern, wobei jeder Behälter eine Querschnittsfläche von nicht größer als 1 cm² aufweist und jeder Samen einer ist, der aufkeimen wird in eine Pflanze der Gattung gewählt aus der Gruppe bestehend aus *Ageratum*, *Arabidopsis*, *Arabis, Artemisia, Asrostis, Browallia, Capsella, Coleus, Cortaderia, Cynodon, Cyperus, Digitalis, Digitaria, Eragrostis, Inula, Ipomopsis, Laevis, Lemna, Nicotiana, Oxalis, Panicum, Petunia, Platycodon, Recta, Sagina, Santonlina, Thymophylla* und *Thymus,* wobei das Verfahren die folgenden Schritte umfasst: Bereitstellen der Samen in wenigstens einem fluidisierten Bett, Bereitstellen einer Vielzahl von vertikalen hohlen Samentransportröhren, wobei jede derartige Röhre an ihrem unteren Ende eine Öffnung aufweist, deren größte Abmessung kleiner als die kleinsten Abmessungen des Samens ist, wobei jede der Röhren eine Einrichtung zum Steuern des Luftdrucks innerhalb des hohlen Abschnitts der Röhre aufweist, wobei der Luftdruck innerhalb jeder Röhre individuell steuerbar ist, Positionieren der Vielzahl der Röhren relativ zu den wenigstens einen fluidisierten Samenbetten derart, dass die unteren Enden der Vielzahl von Röhren teilweise in das wenigstens eine fluidisierte Samenbett eingetaucht sind, Steuern des Luftdrucks innerhalb jeder Röhre, um ein Vakuum innerhalb jeder Röhre zu erzeugen, welches ausreichend zum Einfangen und Halten eines Samens an der Öffnung an dem unteren Ende jeder Röhre ist, Positionieren der Vielzahl von Röhren relativ zu den Behältern derart, dass das untere Ende jeder Röhre innerhalb eines Behälters ist, ungefähr in der Mitte des horizontalen Querschnitts jedes Behälters positioniert, unterhalb des Niveaus des Oberteils des Behälters und über dem Niveau von irgendwelchen Inhalten in dem Behälter, Steuern des Luftdrucks innerhalb jeder Röhre, um das Vakuum zu lösen und einen Druck innerhalb jeder Röhre höher als der Druck außerhalb jeder Röhre, ausreichend zum Lösen und Herauswerfen des Samens in den Behälter, zu erzeugen, und automatisches Erfassen, ob genau ein Samen in einem vorgegebenen Zielgebiet in jedem der Vielzahl von Behältern platziert worden ist, und Aufzeichnen, in welchen Containern ein Samen nicht platziert worden ist.

2. Verfahren nach Anspruch 1, ferner umfassend die folgenden Schritte: Bereitstellen in jedem Behälter, bevor der Samen dorthin ausgeworfen wird, einer Substanz, die dann, wenn sie von dem Samen kontaktiert wird, eine weitere Bewegung des Samens innerhalb des Behälters hemmen oder verhindern wird und hemmen oder verhindern wird, dass der Samen von dem Behälter als Folge von Luftströmungen, die sich von irgendeinem Überschussluftdruck innerhalb der Röhre ergeben, von dem Behälter ausgeworfen wird.

3. Verfahren nach Anspruch 1 oder Anspruch 2, ferner umfassend das Korrigieren der Aufkeimung von irgendeinem Behälter, von dem festgestellt wird, dass er nicht genau einen Samen enthält.

4. Verfahren nach irgendeinem der Ansprüche 1 bis 3, wobei die Querschnittsfläche jedes Behälters nicht größer als 0,5 cm² ist.

5. Vorrichtung zum automatischen Ablegen eines Pflanzensamens oder eines Insekteneis in jeden einer Vielzahl von Behältern, die eine Vielzahl von vertikalen hohlen Samentransportröhren umfassen, wobei jede von derartigen Röhren an ihrem unteren Ende eine Öffnung aufweist, deren größte Abmessung kleiner als die kleinste Abmessung des Samens oder Eis ist, wobei jede der Röhren eine Einrichtung zum individuellen Steuern des Luftdrucks innerhalb des hohlen Abschnitts der Röhre, und eine Einrichtung zum automatischen Erfassen, ob genau ein Samen oder Ei in einem vorgegebenen Zielgebiet in jedem der Vielzahl von Behältern platziert worden ist, und zum Aufzeichnen, in welche Behälter ein Samen oder Ei nicht so platziert worden ist, aufweist.

6. Samentransport zur Verwendung in der Vorrichtung nach Anspruch 5, umfassend eine hohle zylindrische Röhre, die einen äußeren Durchmesser von 1 - 4 mm, ein inneres Loch von 0,5 - 3 mm und eine Öffnung an dem unteren Ende der Röhre aufweist, deren größte Abmessung kleiner als die kleinste Abmessung des Samens ist, und wobei das untere Ende der Röhre eine Abschrägung aufweist, um Samen weg von einem Samen zu lenken, der gerade an der Öffnung gehalten wird.

## Revendications

1. Procédé consistant à déposer automatiquement une graine dans chacun d'une pluralité de conteneurs, chaque conteneur ayant une surface en coupe ne dépassant pas 1 cm² et chaque graine étant une graine qui germera pour devenir une plante de l'espèce sélectionnée dans le groupe suivant : Ageratum, Arabidopsis, Arabis, Artemisia, Asrostis, Browallia, Capsella, Coleus, Cortaderia, Cynodon, Cyperus, Digitalis, Digitaria, Eragrostis, Inula, Ipomopsis, Laevis, Lemna, Nicotiana, Oxalis, Panicum, Pétunia, Platycodon, Recta, Sagina, Santolina, Thymophylla et Thymus, le procédé consistant à placer les graines dans au moins un lit fluidisé, fournir une pluralité de tubes de transport de semis creux verticaux, chacun desdits tubes ayant à son extrémité inférieure une ouverture dont la dimension la plus grande est plus petite que les dimensions de graine les plus petites, chacun desdits tubes ayant des moyens pour contrôler la pression d'air à l'intérieur de la partie creuse du tube, la pression d'air à l'intérieur de chaque tube étant contrôlable individuellement, positionner la pluralité de tubes par rapport à l'au moins un lit de semence fluidisé, de telle sorte que les extrémités inférieures de la pluralité de tubes soient partiellement immergées dans l'au moins un lit de semence fluidisé, contrôler la pression d'air à l'intérieur de chaque tube pour créer un vide à l'intérieur de chaque tube suffisant pour capturer et maintenir une graine au niveau de l'ouverture à l'extrémité inférieure de chaque tube, positionner la pluralité de tubes par rapport aux conteneurs de telle sorte que l'extrémité inférieure de chaque tube soit à l'intérieur d'un conteneur, positionnée approximativement au centre de la section transversale horizontale de chaque conteneur, en dessous du niveau de la partie supérieure du conteneur et en dessous du niveau de n'importe quel contenu dans le conteneur, contrôler la pression d'air à l'intérieur de chaque tube pour relâcher le vide et créer une pression à l'intérieur de chaque tube supérieure à la pression à l'extérieur de chaque tube suffisante afin de relâcher et expulser la graine dans le conteneur, et détecter automatiquement si une et une seule graine est placée dans une zone cible prédéterminée d'une pluralité de conteneurs, et enregistrer les conteneurs dans lesquels aucune graine n'a été placée de la sorte.

2. Procédé selon la revendication 1 comprenant en outre la présence dans chaque conteneur, avant que la graine ne soit expulsée à l'intérieur de celui-ci, d'une substance qui, lorsqu'elle est en contact avec la graine interdit ou empêche tout autre mouvement de la graine à l'intérieur du conteneur et interdit ou empêche que la graine ne soit expulsée du conteneur en raison de courants d'air provoqués par tout excès de pression d'air à l'intérieur du tube.

3. Procédé selon la revendication 1 ou la revendication 2 comprenant en outre la correction de l'ensemencement de tout conteneur qui s'est avéré ne pas contenir une et une seule graine.

4. Procédé selon l'une quelconque des revendications 1 à 3, dans lequel la surface transversale de chaque conteneur n'est pas supérieure à 0,5 cm².

5. Appareil servant à déposer automatiquement une graine de plante ou un oeuf d'insecte dans chacun d'une pluralité de conteneurs comprenant une pluralité de tubes de transport de semis creux verticaux, chacun desdits tubes ayant à son extrémité inférieure une ouverture dont la dimension la plus grande est inférieure à la dimension la plus petite de la graine ou de l'oeuf, chacun desdits tubes ayant des moyens pour contrôler individuellement la pression d'air à l'intérieur de la partie creuse du tube, et des moyens pour détecter automatiquement si une et une seule graine ou un et un seul oeuf a été placé dans une surface cible prédéterminée dans chacun de la pluralité de conteneurs, et pour enregistrer les conteneurs dans lesquels aucune graine ou aucun oeuf n'a été placé de la sorte.

6. Transport de semis devant être utilisé dans l'appareil de la revendication 5 comprenant un tube cylindrique creux ayant un diamètre externe de 1-4 mm, un alésage interne de 0 ; 5-3 mm, ayant une ouverture à l'extrémité inférieure du tube dont la dimension la plus grande est inférieure à la dimension la plus petite de la graine, et l'extrémité inférieure du tube ayant un chanfrein pour dévier les graines d'une graine qui est maintenue au niveau de l'ouverture.
